# EUROPEAN PATENT SPECIFICATION

(11) **EP 1 834 664 B1**
(45) Date of publication and mention of the grant of the patent: **08.05.2013**
(21) Application number: 07004775.8
(22) Date of filing: 08.03.2007
(51) Int. Cl.: A61M 39/26

(54) **Connector**
Verbinder
Connecteur

(30) Priority: 17.03.2006 JP 2006075615
(43) Date of publication of application: 19.09.2007
(73) Proprietor: Covidien AG, 8212 Neuhausen am Rheinfall (CH)
(72) Inventor: Kitani, Ichiro, Fukuroi-shi Shizuoka-ken (JP); Funamura, Shigeaki, Fukuroi-shi Shizuoka-ken (JP); Sakai, Yosuke, Fukuroi-shi Shizuoka-ken (JP)
(74) Representative: Tomlinson, Edward James

(56) References cited:
- EP-A- 1 593 405
- WO-A-96/13301
- WO-A-98/26835
- US-A- 6 063 062

## Description

### Technical field

The present invention relates to a connector that is connected to a liquid distribution means in which is formed a liquid distribution channel. In particular, it relates to a closed-system connector that is normally closed, and opened when necessary to supply liquid to the liquid distribution channel.

### Prior art

In the medical field, transport of fluids, e.g., infusion, blood transfusion, artificial dialysis, or blood collection is often performed. To transport various types of liquids, a tube in the form of a tube body is often used as the liquid distribution means, and a connector is connected to the tube and a plurality of liquids is mixed together or shut off. The connector is attached midway in the tube, and in addition to being used as a coinfusion tool for coinfusing other medicines, it is used in various applications. For example, the function of a switching valve can be provided to the connector, which can be used as a three-way stopcock that controls the supply of liquid from multiple channels, and it can also be used as a terminal connector attached to the end of a medical tube with a normally closed function to normally shut off supply of the liquid from the medical tube and to supply liquid when necessary.

Generally a connector as described above has a channel formation part inside of which is formed a channel space by which a liquid, such as medicine, is distributed. The channel formation part is furnished with a tube body connection opening to which a tube body or the like is connected, the liquid distribution channel in the tube body is connected to the channel space through the tube body connection opening, and the medicine is distributed. A liquid supply means connection opening that connects the liquid supply means for supplying the liquid to be supplied from externally is also formed in the channel formation part. The luer part of a syringe, or the like, as the liquid supply means is inserted into the liquid supply means connection opening, and medicine or the like is supplied into the channel space through the liquid supply means connection opening from the syringe.

However, with a connector in which the liquid supply means connection opening is open, the outside and the channel space are always joined while a luer part is not inserted, and the medicine in the channel space sometimes spills out. There is also the risk of bacterial propagation in areas of the liquid supply means connection opening to which medicine adheres. For this reason, in recent years, closed-system connectors are also used by which a normally closed valve member is attached to the liquid supply means connection opening, and while no luer part is inserted, the liquid supply means connection opening is closed to be liquid-tight.

Japanese Kokai Patent Application No. 2004-16437 describes a closed-system connector with which a valve member attached to a connection opening for an injection needle may be connected and a rubber valve attached by screwing to a cap on top of the valve member. In JP 2004-16437, this connector is described as demonstrating the effect of being able to easily wipe off spilled liquid since a liquid reservoir is produced on the rubber valve when the injection needle is removed from the valve member. EP 1 593 405 A1 discloses a three-port valve in which one port includes a stopper which is compressed around an inner needle tube when a syringe is connected to the port. WO 98/26835 also discloses a valve arrangement having a compressible stopper, the stopper being compressed within an inner chamber on the insertion of a syringe. WO 96/13301 discloses a valve arrangement having similarities to the arrangement of EP 1 593 405. US 6,063,062 discloses a luer arrangement in which a sealing unit is displaced along a central probe on connection thereto, the central probe providing a fluid flow channel therethrough.

### Summary of the invention

With the connector described in JP 2004-16437, an adaptor is provided by which the opening in an injector is disposed at the top of the valve member is pushed in when the injection needle is inserted into the valve member, and the valve member is opened by the adaptor, further pushing the valve member out into the channel in the connector. For this reason, when the injector is connected, the volume of the channel space is decreased by part of the valve member being pushed out into the channel. When the injector is removed in this state, the section pushed out into the channel returns to the original state, and the volume of the channel space increases to return to the original volume. In this case, the pressure in the channel space becomes negative pressure with the increase in volume of the channel space, and there is the risk of the liquid in the channel connected to the channel space flowing backward. Such backflow is undesirable.
The present invention provides a connector according to claim 1. Preferred aspects of the invention are recited in the dependent claims.

The present invention was devised in consideration of such a situation with the objective of providing a connector that can keep the channel space in the connector from going to negative pressure when the liquid supply means is removed, with a closed-system connector.

In order to achieve the abovementioned objective, the features of the connector of the present invention are that, in a connector to which are connected a liquid supply means, inside of which is formed a liquid supply channel, and a liquid distribution means, inside of which is formed a liquid distribution channel, to join the aforementioned liquid supply channel and the aforementioned liquid distribution channel, the connector has: a channel formation part that has a liquid supply means connection opening to which the aforementioned liquid supply means can be connected and a liquid distribution means connection opening to which the aforementioned liquid distribution means can be connected, and inside of which is formed a channel space joined to the aforementioned liquid supply means connection opening and the aforementioned liquid distribution means connection opening; a valve member that is attached to the aforementioned liquid supply means connection opening, that has a moving part that moves with pressure imparted by the aforementioned liquid supply means when the aforementioned liquid supply means is connected to the aforementioned liquid supply means connection opening, and that opens due to the aforementioned pressure; and an accommodating chamber that is furnished outside the aforementioned channel space and that accommodates the aforementioned moving part.

With the connector of the present invention constituted as described above, the valve member has a moving part that is pressed by the liquid supply means and moves when a liquid supply means is connected to the liquid supply means connection opening, the valve member. The moving part is accommodated in an accommodating chamber furnished outside the channel space, so the aforementioned moving part is prevented from protruding into the channel space and decreasing the channel volume. For this reason, even when the liquid supply means is removed from the opening, increase in the channel volume can be restrained to prevent its going to negative pressure, and thus backflow of the liquid flowing in the channel joined to the channel space can be prevented.

The aforementioned connector may be anything that can connect a liquid supply means and a liquid distribution means. For example, it could be used as a coinfusion tool for coinfusing another liquid into a channel, or it could be used as a three-way stopcock whereby the connector is provided with a switching valve function to control the supply of liquids from multiple channels. It could also be used as a terminal connector attached to the end of a medical tube with a normally closed function to shut off supply of the liquid from the medical tube and to supply liquid when necessary.

It is sufficient if the aforementioned accommodating chamber accommodates the moving part to affect channel space volume change within the moving part. Therefore, it need not accommodate a part that is deformed and moves within the external form of the original valve member. The aforementioned accommodating chamber could also be formed integrally with the channel formation part, or it could be formed separately. When formed integrally, a constitution in which a channel space and an accommodating space are formed delineated within one housing is better.

The aforementioned accommodating chamber may have a window part that connects to the outside. When the liquid supply means is attached to or pulled out of the liquid supply means connection opening, the moving member advances or retreats in the accommodating chamber and the volume of the space in the accommodating chamber changes. In this case, by providing a window part in the accommodating chamber, the pressure in the accommodating chamber may be kept constant even if the volume of the space in the accommodating chamber changes. Thus, difficulty in accommodating the moving part in the accommodating chamber or difficulty in withdrawing the moving part from the accommodating chamber can be prevented.

The aforementioned valve member is such that a slit is formed in the side facing the channel space. In addition, the aforementioned moving part has a first moving part and a second moving part formed by expanding to the two sides of the aforementioned slit when the aforementioned liquid supply means is connected to the aforementioned liquid supply means connection opening, and the aforementioned accommodating chamber has a first accommodating chamber that accommodates the aforementioned first moving part and a second accommodating chamber that accommodates the aforementioned second moving part. With a constitution such as this, the first moving part and the second moving part can easily be accommodated in the first accommodating chamber and the second accommodating chamber by the valve body splitting left and right relative to the slit. In this case, it is even better if the aforementioned slit penetrates from the outside to the aforementioned channel space. By the slit penetrating from the outside to the channel space, the liquid supply channel can be joined to the channel space through the slit when a liquid supply means is connected to the liquid supply means connection opening.

The aforementioned connector also has an auxiliary valve member that joins or disconnects the valve placement space at which the aforementioned valve member is placed and the aforementioned channel space. The auxiliary valve member closes prior to the aforementioned valve member and disconnects the aforementioned channel space and the aforementioned valve placement space when the aforementioned liquid supply means is pulled from the aforementioned liquid supply means connection opening. Since the channel space and the valve placement space are disconnected by the closing of the auxiliary valve member, a change in volume accompanying the closing of the valve member subsequently is not transmitted to the channel space. Thus, even a minute change in volume accompanying closing of the valve member can be prevented from being transmitted to the channel space, and backflow in the liquid distribution channel joined to the channel space can be prevented even more completely.

### Brief description of the figures

Figure 1 is a plan view of a three-way stopcock pertaining to a first embodiment of the present invention.
Figure 2 is a plan view of a three-way stopcock pertaining to a first embodiment of the present invention.
Figure 3 is a plan view of a three-way stopcock pertaining to a first embodiment of the present invention.
Figure 4 is a cross section along A-A in Figure 1.
Figure 5 is a cross section along B-B in Figure 2
Figure 6 is an oblique view of a valve body pertaining to a first embodiment of the present invention.
Figure 7 is an exploded oblique view of a valve body pertaining to a first embodiment of the present invention.
Figure 8 shows operation when the valve body furnished for a connector pertaining to a first embodiment of the present invention is opened and closed; (a) shows the state before the luer part pushes against the valve body, (b) shows the state when the luer part is pushing against the valve body, and (c) shows the state when the luer part has penetrated the slit in the valve body and it is opened.
Figure 9 shows the operation of a valve body and an auxiliary valve furnished in a connector pertaining to a second embodiment of the present invention when they are opened and closed; (a) shows the state before the luer part pushes against the valve body, (b) shows the state when the luer part is pushing against the valve body, (c) shows the state when the luer part has penetrated the slit in the valve body but the auxiliary valve body has not been pushed, and (d) shows the state when the luer part has pushed against the auxiliary body and has opened it.
Figure 10 is a plan view of a coinfusion tool to which the present invention is applied.
Figure 11 is a front view of a coinfusion tool to which the present invention is applied.
Figure 12 is a left side view of a coinfusion tool to which the present invention is applied.
Figure 13 is a cross section along C-C in Figure 10.
Figure 14 is a cross section along D-D in Figure 11.
Figure 15 is a plan view of a terminal connector to which the present invention is applied.
Figure 16 is a front view of a terminal connector to which the present invention is applied.
Figure 17 is a left side view of a terminal connector to which the present invention is applied.
Figure 18 is a cross section along E-E in Figure 15.
Figure 19 is a cross section along F-F in Figure 16.
Figure 20 is a cross section of a three-way stopcock pertaining to a third embodiment of the present invention; (a) shows the state when the valve body is opened, and (b) shows the state when the valve body is closed.

### Detailed description of preferred embodiments of the invention

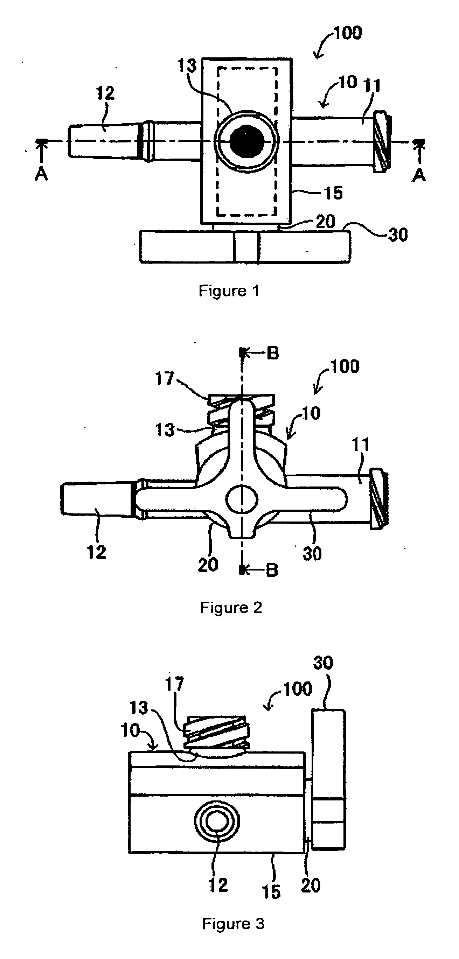

Below, a connector pertaining to the present invention will be explained in detail using figures. As the first embodiment, an example in which a connector pertaining to the present invention is used as a three-way stopcock will be explained with figures. Figure 1 is a plan view of a three-way stopcock pertaining to the first embodiment of the present invention, Figure 2 is a front view, and Figure 3 is a side view with Figure 2 as the front view. As can be seen from these figures, three-way stopcock (100) has a housing (10) that forms its contours, a stopper body (20) mounted in housing (10), and a grip part (30) formed integrally with stopper body (20).

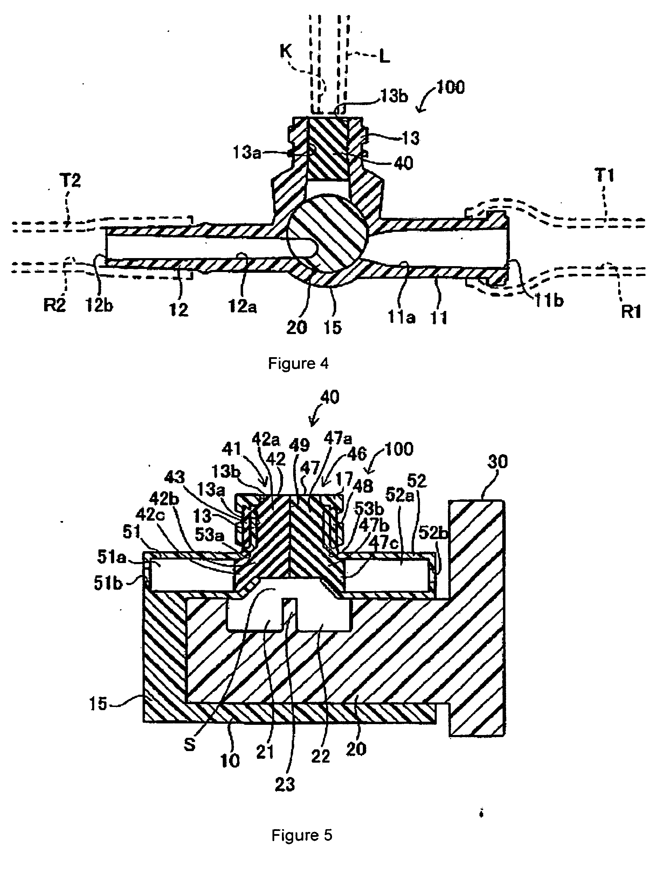

Housing (10) has a tubular part (15) and a first branch tube (11), a second branch tube (12) and a third branch tube (13), which are three branch tubes attached to tubular part (15). A round columnar space is formed inside tubular part (15). Figure 4 is a cross section along A-A in Figure 1. As can be seen from Figure 4, a branch channel (first branch channel (11a), second branch channel (12a) and third branch channel (13a)) is formed in the respective branch tubes (11), (12) and (13), and each branch channel (11 a), (12a) and (13a) opens into the round columnar space formed inside tubular part (15). A valve body (40), described below, is also attached to third branch channel (13a). Tubular part (15), first branch tube (11), second branch tube (12) and third branch tube (13) correspond to the channel formation port in the present invention. Here, in this embodiment, polycarbonate (PC) is used as the material for housing (10), but polypropylene (PP), polyethylene terephthalate (PET) or another resin material may be used satisfactorily.

Figure 5 is a cross section along B-B in Figure 2. As shown in Figures 4 and 5, stopper body (20) has a round columnar shape, and it is fit to be able to rotate inside the round columnar space formed around the inner circumference of tubular part (15). Also, as shown in Figure 5, slots (21) and (22) are formed around the outer circumference of tubular part. The shape of slots (21) and (22) is designed to be able to join with any of branch channels (11 a), (12a) and (13a) by the turning of stopper body (20). A partition (23) is formed between slots (21) and (22). It is made so that when liquid inside slot (21) overflows partition (23) it flows into slot (22). One end of stopper body (20) protrudes from housing (10) and a grip part (30) is attached to the protruding end. Grip part (30) has three arms (refer to Figure 2) and it can turn integrally with stopper body (20). Therefore, by turning grip part (30), stopper body (20) also turns inside the round columnar space in tubular part (15). The way in which slots (21) and (22) are placed changes according to the turning of stopper body (20) and joining or connection of branch channels (11 a), (12a) and (13a) can be switched. Here, for the material for stopper body (20) and grip part (30), polyethylene (PE) is used, but polyoxymethylene (POM), polypropylene (PP) or another resin can also be used.

As can be seen from Figure 4, first branch channel (11 a) is joined from the right side in the figure to the side of the round columnar space in tubular part (15) and an open part (11 b) is formed at the right in the figure. Second branch channel (12a) is also connected from the left side in the figure to the side of the aforementioned round columnar space, and an open part (12b) is formed at the left in the figure. In addition, third branch channel (13a) is joined from the top in the figure to the side of the aforementioned round columnar space and an open part (13b) is formed at the top in the figure. One end of a medical tube (T1) as the liquid supply means, inside of which is formed a liquid distribution channel (R1) by which liquid is distributed, can be connected to open part (11 b) of first branch channel (11). One end of medical tube (T2) as the liquid distribution means, inside of which is formed a liquid distribution channel (R2) by which liquid is distributed, can be connected to open part (12b) of second branch channel (12a). The luer part (L) of a syringe as a liquid supply means, inside of which is formed a liquid supply channel (K) for supplying liquid to three-way stopcock (100), can be connected to open part (13b) of third branch channel (13a).

As shown in Figures 2 and 4, first branch tube (11), second branch tube (12) and third branch tube (13) are connected to tubular part (15) at 90 degree intervals. First branch tube (11) and second branch tube (12) are disposed facing on two sides of tubular part (15). Third branch tube (13) is disposed in a position at a 90 degree interval from both first and second branch channels [sic; tubes] (11) and (12) in the circumferential direction of tubular part (15), and third branch channel (13a) is formed to intersect first branch channel (11a) and second branch channel (12a) at a right angle.

As shown in Figure 5, housing (10) is furnished with a first valve body accommodating chamber (51) and a second valve body accommodating chamber (52) above tubular part (15) in the figure. First valve body accommodating chamber (51) and second valve body accommodating chamber (52) are formed extending to the left and right in the figure below third branch tube (13), and a semicylindrical shaped valve body accommodating space (51a) and (52a) is formed inside each, respectively. A window part (51b) and (52b) is formed at one end of first and second valve body accommodating chambers (51) and (52), and first and second valve body accommodating spaces (51a) and (52b) are connected to the outside through the window parts (51b) and (52b). At the other end of first and second valve body accommodating chambers are formed guide channels (53a) and (53b) formed extending upward diagonally as shown in Figure 5. Third branch tube (13) is connected at the top ends of guide channels (53a) and (53b).

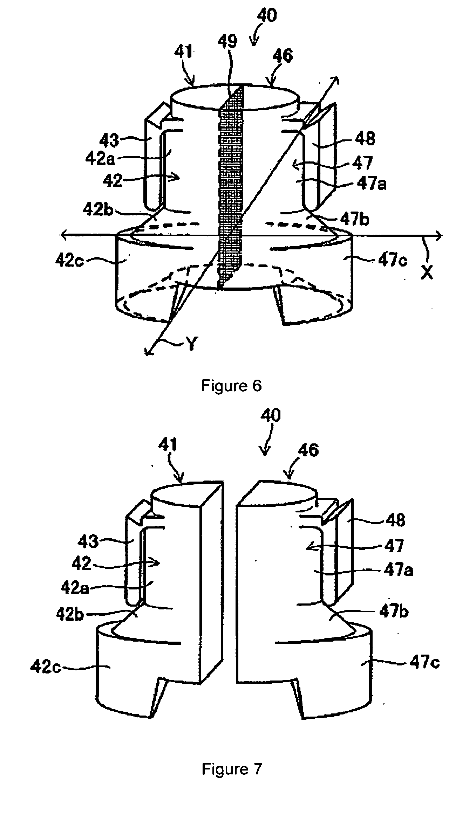

As shown in Figures 4 and 5, a valve body (40) is attached to third branch channel (13a). Valve body (40) is an open-close valve that is normally closed, and in the state shown, is closed and open part (13b) is closed. Figure 6 is an oblique view of valve body (40) and Figure 7 is an exploded oblique view of valve body (40). As shown in these figures, valve body (40) is constituted a pair of valve body pieces (first valve body piece (41) and second valve body piece (46)) in a symmetrical shape, and first and second valve body pieces (41) and (46) are attached to open part (13b) of third branch channel (13a) facing each other symmetrically. The two valve body pieces (41) and (46) have main bodies (42) and (47) and support arms (43) and (48), respectively. Main bodies (42) and (47), when the two valve body pieces (41) and (46) are assembled as shown in Figure 6, have a round columnar part (42a) and (47a) formed in a round columnar shape from the top, a tapered part (42b) and (47b) that increases in diameter in a tapered shape at the bottom in the figure of round columnar parts (42a) and (47a), and large-diameter parts (42c) and (47c) that further increase in diameter horizontally from the bottom ends in the figure of tapered parts (42b) and (47b) and that that are also formed in a cylindrical shape downward vertically. The inside of large-diameter parts (42c) and (47c) is bored into a truncated cone shape from the bottom end.

In this embodiment, tapered parts (42b) and (47b) do not increase in diameter in the same direction but rather increase in diameter in a specific diametral direction (direction of the X axis in Figure 6) and do not increase in diameter in the diametral direction perpendicular to that direction (the direction of the Y axis in Figure 6). Therefore, tapered parts (42b) and (47b) and large-diameter parts (42c) and (47c) have an elliptical shape when viewed from the top surface. Valve body (40) with such a shape is cut vertically in the Y direction in Figure 6 to give a pair of valve body pieces (41) and (46). Therefore, the surfaces at which the two valve body pieces (41) and (46) face are planar, and a slit (49) that penetrates through valve body (40) from the top to the bottom in the figure is formed by the facing of the planes.

As shown in Figures 6 and 7, support arms (43) and (48) are attached on the side circumference of round columnar parts (42a) and (47a). Support arms (43) and (48) are furnished in the direction of the X axis in Figure 6 to be symmetrical relative to aforementioned slit (49). Support arms (43) and (48) are formed thin to be able to bend and are formed extending toward the outside diametrally from the side circumference of round columnar parts (42a) and (47a).

Valve body (40) with the aforementioned constitution is attached to third branch channel (13a) as shown in Figure 5 with the pair of valve body pieces (41) and (46) assembled. In this case, round columnar parts (42a) and (47a) are disposed on third branch channel (13a) tapered parts (42b) and (47b) are disposed in guide channels (53a) and (53b) connected to third branch tube (13), and large-diameter parts (42c) and (47c) are disposed to slightly enter first valve body accommodating chamber (51) and second valve body accommodating chamber (52) that connect to guide channels (53a) and (53b). When valve body (40) is disposed in this way, slit (49) is formed to penetrate from the outside to channel space (S). Open part (13b) in third branch channel (13a) is also covered by a cap (17). Cap (17) has a ring shape with a hole in the inner circumference, and threads for luer locking are formed on the outside surface (refer to Figures 2 and 3). Support arms (43) and (48) are bent by cap (17) by cap (17) being secured covering aforementioned open part (13b) and the inner surface of the side circumference of cap (17) and the outside surface of the side circumference of third branch tube (13) are tightly secured together.

In three-way stopcock (100) of this embodiment that is constituted as described above, as shown in Figure 4, grip part (30) is turned while medical tubes (T1) and (T2) are attached to open part (11b) of first branch tube (11) and open part (12b) of second branch tube (12) to join first branch channel (11a) to slot (21) formed in stopper body (20) and join second branch channel (12a) to slot (22). Then medicine is supplied from medical tube (T1) connected to first branch tube (11). By so doing, the medicine flows into slot (21) of stopper body (20) from first branch channel (11 a). The medicine in slot (21) spills over partition (23) and enters slot (22). Then it flows into second branch channel (12a) from slot (22). A channel is formed in this way. Here, the space in slots (21) and (22), the space above partition (23), and first and second branch channels (11 a) and (12a) constitute channel space (S) in three-way stopcock (100).

In this case, open part (13b) in third branch tube (13) as shown in Figure 5 is blocked in a liquid-tight manner valve body (40) and closed. Therefore, the liquid flowing through channel space (S) does not leak outside from third branch tube (13). No impurities will enter third branch channel (13a) through open part (13b) in third branch tube (13) from the outside either.

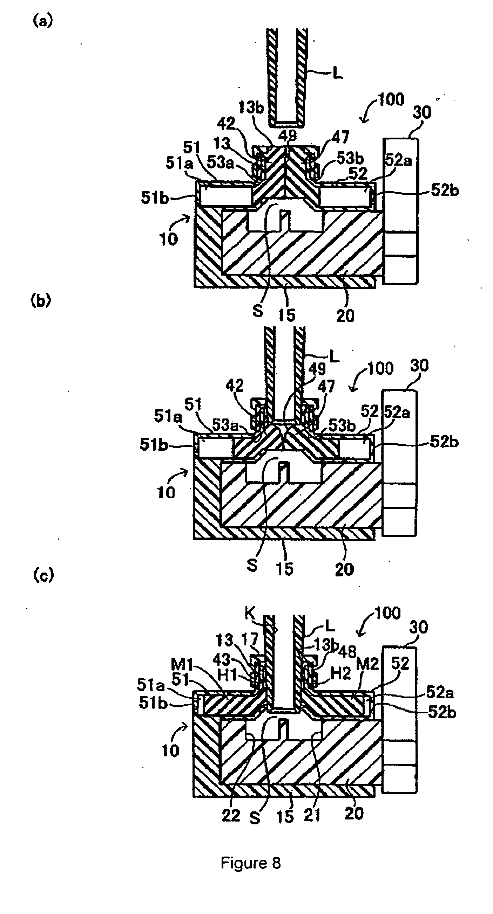

Figures 8 (a)-(c) show the operating state when valve body (40) is opened and closed. To connect luer part (L) to open part (13b) of third branch tube (13), first, as shown in Figure 8 (a), the tip of luer part (L) is brought near the top surface of main bodies (42) and (47) of valve body (40). Then the tip of luer part (L) is pushed against main bodies (42) and (47) from the top surface and main bodies (42) and (47) are pushed into third branch tube (13). By so doing, the top of slit (49) is pushed open by the pressing force from luer part (L) as shown in Figure 8 (b) and main bodies (42) and (47) also elastically deform. Main bodies (42) and (47) are accommodated in first and second valve body accommodating chambers (51) and (52) through guide channels (53a) and (53b) by this elastic deformation.

When main bodies (42) and (47) are further pushed into third branch tube (13) by luer part (L), as shown in Figure 8 (c), slit (49) opens vertically and luer part (L) is exposed to channel space (S). Then, main bodies (42) and (47) enter first and second valve body accommodating chambers (51) and (52). After the state as shown in Figure 8 (c) is reached, a lock ring (not shown) in which luer part (L) is inserted is secured by screwing using threads formed on the outer circumference of cap (17) so that attachment (luer access) of luer part (L) to open part (13b) of third branch tube (13) is completed. Here, not only a syringe but a medical tube can also be connected to luer part (L).

When luer access is completed, valve body (40) is in the state as shown in Figure 8 (c). Main body (42) deforms to form a part that (first moving part (M1)) moves into first valve body accommodating chamber (51) and a part that is drawn out (first drawn-out part (H1)) between luer part (L) and third branch tube (13) by the pressing force from luer part (L), and these parts are supported by support arm (43). In the same way, main body (47) deforms to form a part (second moving part (M2)) that moves into second valve body accommodating chamber (52) and a part that is drawn out (second drawn-out part (H2)) between luer part (L) and third branch tube (13) by the pressing force from luer part (L), and these parts are supported by support arm (48). When medicine is supplied from a syringe or the like connected to luer part (L) in this state, the medicine is injected into slot (21) or (22) from liquid supply channel (K) and is mixed into the liquid flowing through channel space (S). Medicine from third branch tube (13) is coinfused in this way. Here, three-way stopcock (100) of this embodiment can switch channel connects by the turning of stopper body (20). For example, second branch channel (12a) and third branch channel (13a) can be joined, and first branch channel (11a) and second branch channel (12a) can be disconnected. Luer part (L) can be connected to open part (13b) in third branch tube (13) and a liquid from luer part (L) can be supplied to second branch channel (12a) in such a state.

When luer part (L) is pulled out (luer removal) from open part (13b) of third branch tube (13), after luer part (L) is unsecured, luer part (L) is lifted upward in Figure 8 (c). By so doing, first and second moving parts (M1) and (M2) that are accommodated in first and second valve body accommodating chambers (51) and (52) withdraw from accommodating chambers (51) and (52) due to their restorative force, and the section that has become a space due to luer part (L) being pulled out is covered. In this case, slit (49) closes starting from the farthest section from the tip of luer part (L), that is, the section facing channel space (S), as shown in Figure 8 (b), and the closed section rises as luer part (L) rises. Then, when luer part (L) is completely pulled out, the state in Figure 8 (a) is restored.

As above, with three-way stopcock (100) of this embodiment, first and second valve body accommodating chambers (51) and (52), in which are accommodated first and second moving parts (M1) and (M2) that move due to the pressing force from luer part (L), are formed in valve body (40), so first and second moving parts (M1) and (M2) do not enter channel space (S) during luer access or luer removal. Thus, the movement of first and second moving parts (M1) and (M2) does not affect the change in volume of channel space (S) during luer removal, and channel space (S) can be kept from reaching negative pressure by the aforementioned movement. Also, during luer removal, slit (49) closes starting from the section most distant from the tip of luer part (L), that is, the section facing channel space (S), as shown in Figure 8 (b), so the change in volume in channel space (S) that accompanies opening and closing of slit (49) can be kept to a minimum, the change in pressure inside channel space (S), particularly channel space (S) reaching negative pressure, can be controlled even better. For this reason, the liquid in liquid distribution channels (R1) and (R2) in medical tubes (T1) and (T2) connected to three-way stopcock (100) can be kept from flowing backward.

Window parts (51b) and (52b) are also formed in first and second valve body accommodating chambers (51) and (52) and first and second valve body accommodating spaces (51a) and (52a) are joined with the outside through window parts (51b) and (52b). So, pressure change in first and second valve body accommodating spaces (51a) and (52a) when first and second moving parts (M1) and (M2) are withdrawn inside first and second valve body accommodating chambers (51) and (52) can be controlled. Thus, positive pressure can be prevented from occurring inside first and second valve body accommodating spaces (51a) and (52a) and valve body (40) from being pushed in during luer access. Negative pressure can also be prevented from occurring in first and second valve body accommodating spaces (51 a) and (52a) so that luer part (L) cannot be pulled out during luer removal. Guide channels (53a) and (53b) are also furnished between first and second valve body accommodating chambers (51) and (52) and third branch tube (13), so main bodies (42) and (47) can enter first and second valve body accommodating chambers (51) and (52) smoothly through guide channels (53a) and (53b) and the reliability of the operation to open valve body (40) can be improved.

Next, a second embodiment of the present invention will be explained. This embodiment is characterized in being furnished with a flat plate-like auxiliary valve body. In other aspects, the constitution is the same as that of the aforementioned first embodiment.

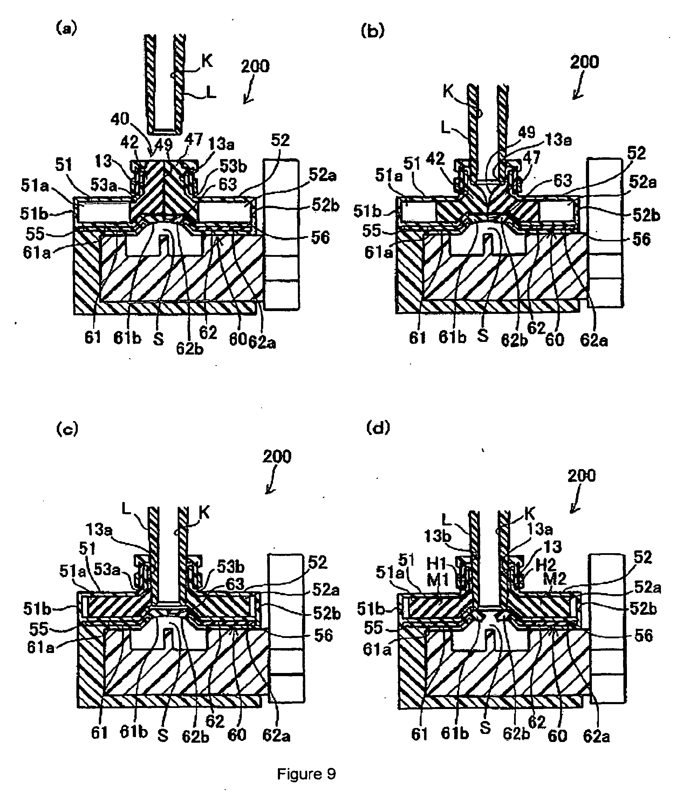

Figures 9 (a) - 9 (d) show the structure of a three-way stopcock (200) in this embodiment and the operation when valve body (40) is opened and closed. As shown in Figure 9 (a), three-way stopcock (200) in this embodiment has first and second auxiliary valve body accommodating chambers (55) and (56) directly below first and second valve body accommodating chambers (51) and (52), respectively. Flat plate-like auxiliary valve body pieces (61) and (62) are disposed in first and second auxiliary valve body accommodating chambers (55) and (56), respectively. First and second auxiliary valve body pieces (61) and (62) are paired to constitute one auxiliary valve body (60). First and second auxiliary valve body pieces (61) and (62) have fixed parts (61 a) and (62a) disposed in first and second auxiliary valve body accommodating chambers (55) and (56), and valve parts (61b) and (62b) connected to fixed parts (61a) and (62a) and also placed in channel space (S). The tips of valve parts (61 b) and (62b) contact each other, and a slit (63) is formed by this contact. Slit (63) is formed directly below slit (49) in valve body (40); it is normally closed, and is made to open with pressing force. Auxiliary valve body (60) disposed in this way is constituted to join or disconnect third branch channel (13a), which is the valve placement space in which valve body (40) is placed, and channel space (S). Otherwise, the constitution is the same as that of the aforementioned first embodiment.

In three-way stopcock (200) with the aforementioned constitution, when luer part (L) is pushed into third branch tube (13), valve body (40) deforms as shown in Figure 9 (b) and main bodies (42) and (47) go into first and second valve body accommodating chambers (51) and (52). When luer part (L) is further pushed into the third branch tube, luer part (L) penetrates slit (49) as shown in Figure 9 (c) and slit (49) opens. However, in the state in Figure 9 (c), the tip of luer part (L) does not contact auxiliary valve body (60), so slit (63) formed in auxiliary valve body (60) remains closed. When luer part (L) is pushed slightly downward from this state, the tip of luer part (L) contacts auxiliary valve body (60) as shown in Figure 9 (d). For this reason, valve parts (61 b) and (62b) of auxiliary valve body (60) are pushed by luer part (L) and slit (63) opens. Both valve body (40) and auxiliary valve body (60) are opened by this and liquid supply channel (K) in luer part (L) is joined to channel space (S). Luer access is completed in this way.

During luer removal, luer part (L) is pulled out upward in Figure 9 (d). By so doing, slit (63) in auxiliary valve body (60) first opens, giving the state in Figure 9 (c). In this state, liquid supply channel (K) of luer part (L) and channel space (S) are already disconnected. When luer part (L) is lifted further, the state in Figure 9 (b) returns to the state shown in Figure 9 (a). Luer removal is completed in this way.

With this embodiment as above, an auxiliary valve body (60) that joins or disconnects third branch channel (13a), which is the valve placement space in which valve body (40) is placed, and channel space (S) closes prior to valve body (40) during luer removal to disconnect third branch channel (13a) and channel space (S). For this reason, the closing of valve body (40) during luer removal can be prevented from affecting the change in volume of channel space (S), and channel space (S) can be even more effectively kept from reaching negative pressure. In this case, valve parts (61b) and (62b) that close auxiliary valve body (60) are disposed in channel space (S) and are positioned directly below slit (49) in valve body (40). Thus, even when valve parts (61 b) and (62b) close, there is no change in volume in channel space (S) before and after the closing. Thus, channel space (S) does not reach negative pressure in conjunction with the closing of auxiliary valve body (60).

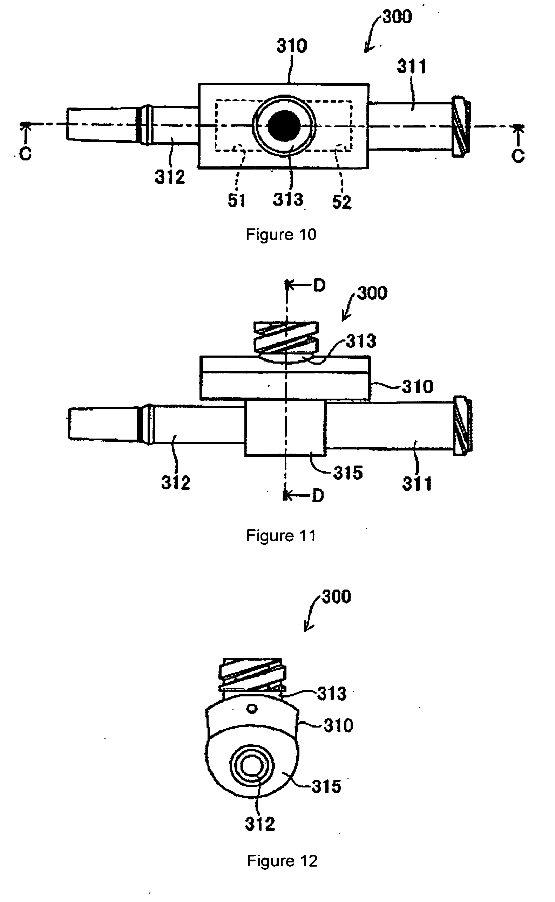

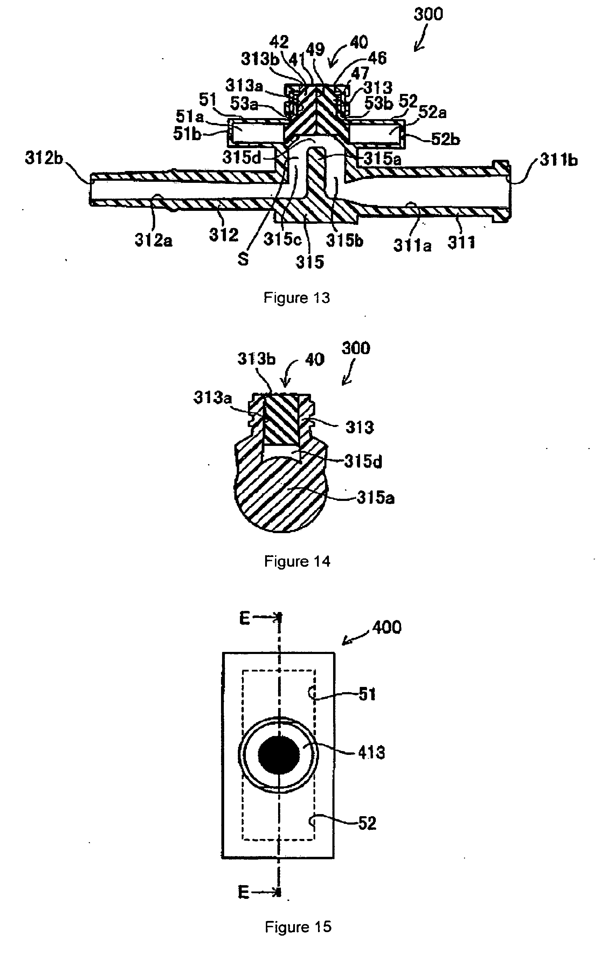

With the aforementioned first and second embodiments, examples in which the connector of the present invention was applied to a three-way stopcock, but the present invention can also be applied to a coinfusion tool for coinfusing a liquid in the middle of a channel. Figure 10 is a plan view of a coinfusion tool to which the present invention is applied, Figure 11 is a front view, Figure 12 is a left side view when Figure 11 is a front view, Figure 13 is a cross section along C-C in Figure 10, and Figure 14 is a cross section along D-D in Figure 11.

As can be seen from the figures, coinfusion tool (300) has a housing (310) that forms its contours. Housing (310) has a first branch tube (311), a second branch tube (312), a third branch tube (313) and a main body (315), and branch tubes (311), (312) and (313) are each connected to main body (315). Their respective branch channels (first branch channel (311a), second branch channel (312a) and third branch channel (313a)) are formed in branch tubes (311), (312) and (313). As shown in Figure 13, inside main body (315) are formed a first joining space (315b) and a second joining space (315c). A partition (315a) is formed between first joining space (315b) and second joining space (315c). First joining space (315b) and second joining space (315c) can be joined through upper space (315d) positioned at the top of partition (315a) in the figure.

First joining space (315b) is joined with first branch channel (311a) and second joining space (315c) is joined with second branch channel (312a). Therefore, first branch channel (311a) is joined with second branch channel (312a) through first joining space (315b), upper space (315d) and second joining space (315c). In this example, channel space (S) in coinfusion tool (300) is constituted with first branch channel (311a), second branch channel (312a), first joining space (315b), second joining space (315c) and upper space (315d).

As can be seen from Figure 13, an open part (311 b) in first branch channel (311 a) is formed to the right in the figure. Open part (311 b) can be connected with a medical tube or the like as a liquid supply means. An open part (312b) in second branch channel (312a) is formed at the left in the figure. A medical tube or the like as a liquid supply means can also be connected to open part (312b). An open part (313b) in third branch channel (313a) is formed at the top in the figure. The luer part or the like of a syringe or the like as a liquid supply means can be connected to open part (313b).

As shown in Figures 13 and 14, a valve body (40) is attached to open part (313b) in third branch channel (313a). A first valve body accommodating chamber (51) and a second valve body accommodating chamber (52) are also furnished integrally horizontally below third branch tube (313) in the figure. First and second valve body accommodating chambers (51) and (52) are joined to third branch tube (313) through guide channels (53a) and (53b). The actual constitution of valve body (40), first valve body accommodating chamber (51), second valve body accommodating chamber (52) and guide channels (53a) and (53b) is the same as the constitution explained in the aforementioned first embodiment.

In coinfusion tool (300) of this embodiment that is constituted as described above, medicine is supplied to a medicine tube connected to first branch tube (311). By so doing, the medicine flows from first branch channel (311 a) to first joining space (315b) of main body (315). The medicine in first joining space (315b) spills over partition (315a) and enters upper chamber (315d). The medicine further goes on both sides of partition (315a) from upper space (315d) and enters second joining space (315c) formed on the opposite side from first joining space (315b). Then it flows from second joining space (315c) to second branch channel (312a). A flow is formed in this way.

When medicine is coinfused from third branch tube (313), the luer part of a syringe is positioned at the top surface of valve body (40) and valve body (40) is pushed into third branch tube (313). The operation of valve body (40) during luer access and luer removal is the same as the operation explained following Figure 8 in the aforementioned first embodiment. In this way, the connector of the present invention can also be applied to a coinfusion tool.

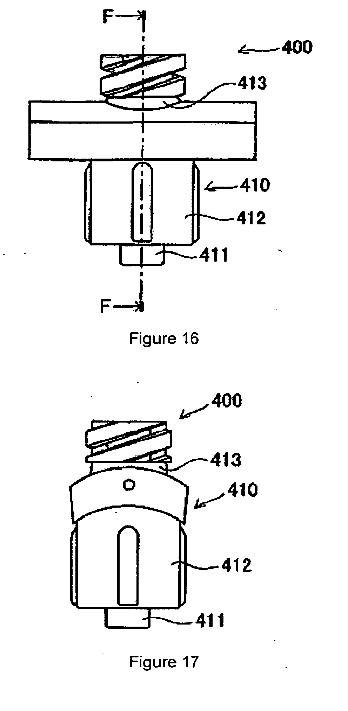

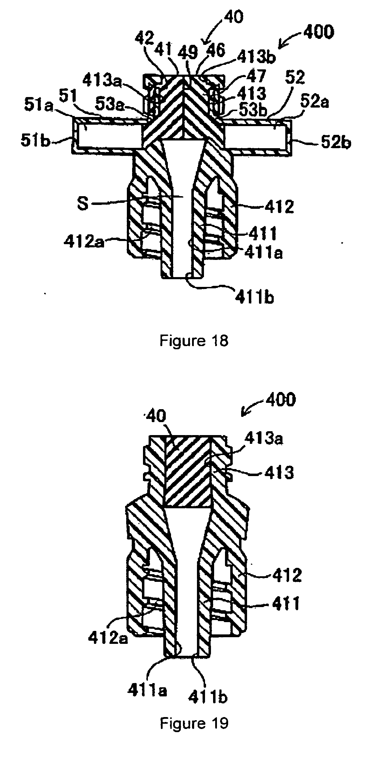

The connector of the present invention can also be applied as a terminal connector that is connected to the end of a channel, that normally closes the channel, and that opens the channel when a tube or a syringe luer part is attached. Figure 15 is a plan view of a terminal connector to which the present invention is applied, Figure 16 is a front view, Figure 17 is a left side view when Figure 16 is a front view, Figure 18 is a cross section along E-E in Figure 15, and Figure 19 is a cross section along F-F in Figure 16.

As can be seen from the figures, terminal connector (400) of this embodiment has a housing (410) that forms its contours. A male connection part (411), a cover part (412) and a terminal connection part (413) are formed in housing (410).

Male connection part (411) has a long, narrow cylindrical shape and a channel (411a) is formed on the inside. From open part (411b) of channel (411a), a female connection part attached to the terminus of a liquid supply means, such as a tube, is connected. Cover part (412) is formed in a cylindrical shape to cover the outer circumference of male connection part (411). An inner thread (412a) is also formed on the inner wall of cover part (412). Inner thread (412a) screws into a male threaded member attached to the tube connected to male connection part (411) and is used to secure a female connection part or the like. Terminal connection part (413) is joined to the end of male connection part (411), and a valve placement space (413a) in which valve body (40) is placed is formed inside. Valve placement space (413a) is joined to channel (411a) in male connection part (411) as shown in the figures, and one end is open because of opening (413b). Open part (413b) can be connected with a liquid supply means such as the luer part of a syringe. Here, channel (411a) corresponds to channel space (S) in the present invention.

As shown in Figures 18 and 19, a valve body (40) is attached to open part (413b) of valve placement space (413a). A first valve body accommodating chamber (51) and a second valve body accommodating chamber (52) are furnished integrally and horizontally below terminal connection part (413) in the figure. First and second valve body accommodating chambers (51) and (52) are joined to terminal connection part (413) through guide channels (53a) and (53b). The actual constitution of valve body (40), first valve body accommodating chamber (51), second valve body accommodating chamber (52) and guide channels (53a) and (53b) is the same as the constitution explained with the aforementioned first embodiment.

In a terminal connector (400) constituted as described above, first, a female connection part or the like (not shown) attached to the terminus of a tubular body, such as a tube, is connected to male connection part (411) and the female connection part is secured by screwing using inner thread (412a) of cover part (412). In this case, when a liquid supply means, such as the luer part of a syringe, is attached to valve body (40), open part (413b) of terminal connection part (413) is blocked in a liquid-tight manner by valve body (40) and is closed as shown in Figure 18. When medicine is supplied from terminal connection part (413), the luer part of a syringe or the like is positioned at the top surface of valve body (40) and valve body (40) is pushed into terminal connection part (413). The operation of valve body (40) during luer access and luer removal is the same as the operation explained following Figure 8 in the aforementioned first embodiment. In this way, the present invention can also be used as a terminal connector.

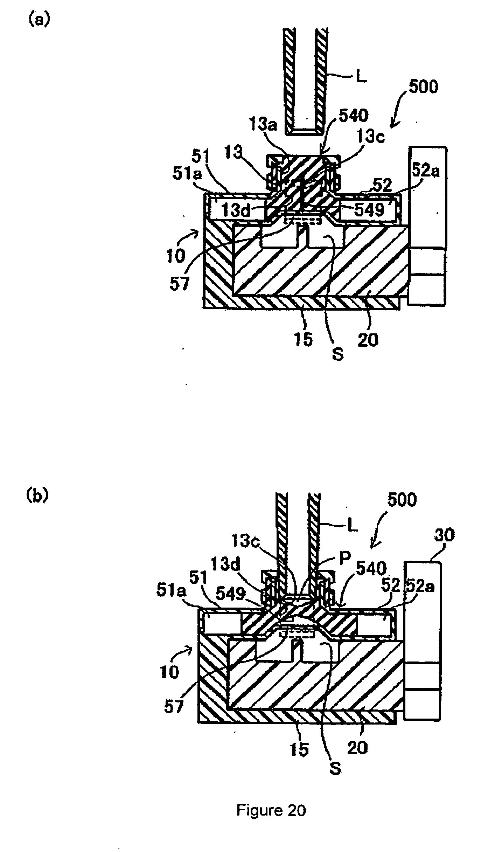

Figure 20 is a cross section showing a third embodiment of a connector of the present invention; (a) shows the state before valve body (540) is opened and (b) shows the state when valve body (540) has been opened. As can be seen from Figure 20 (a), the connector of this embodiment is a three-way stopcock. The basic constitution of the three-way stopcock (500) is the same as three-way stopcock (100) explained in the aforementioned first embodiment, so the same sections below are represented with the same symbols and the explanation will concentrate on the differences. Three-way stopcock (500) has a valve body (540). A slit (549) is formed in valve body (540). Slit (549) is formed at the height of around 2/3 from the bottom end of valve body (540), it is formed at the side facing channel space (S), and it does not penetrate up and down. For this reason, valve body (540) is formed in one piece. The structure of valve body (540) otherwise is the same as the structure of valve body (40) explained with the aforementioned first embodiment. Third branch channel (13a) and first and second valve body accommodating chambers (51) and (52) are joined with or disconnected from channel space (S) by joining wall (57). In addition, in the side wall of third branch tube (13) is formed a through hole (13c) in an inverted triangular shape. A joining path (13d) that joins third branch channel (13a) and channel space (S) is formed from through hole (13c). Otherwise, the constitution is the same as the aforementioned first embodiment.

In three-way stopcock (500) with the aforementioned constitution, during luer access, valve body (540) is pushed into third branch tube (13) by pushing luer part (L) against the top surface of valve body (540). Slit (549) formed in valve body (540) opens left and right by the aforementioned pressing force. For this reason, valve body (540) is expanded to two sides bordering on slit (549), one of the expanded sections enters first valve body accommodating chamber (51) and the other enters second valve body accommodating chamber (52). A force acts whereby the top surface of valve body (540) bends in to the center due to slit (549) opening. For this reason, as shown in Figure 20 (b), the top surface of valve body (540) bends and a depression (P) is formed. Aforementioned through hole (13c) is formed near where depression (P) is formed. Therefore, the space in depression (P) is joined to channel space (S) from aforementioned through hole (13c) through joining path (13d). In this case, deformation in valve body (540) accompanying the opening of valve body (540) is accomplished by first and second valve body accommodating spaces (51 a) and (52a) that are not joined with channel space (S), so the volume of channel space (S) does not change accompanying the opening of valve body (540).

During luer removal, the sections that have entered first and second valve body accommodating spaces (51a) and (52a) withdraw due to their restorative force and depression (P) formed in then top surface of valve body (540) also disappears. For this reason, luer part' (L) is disconnected from channel space (S). In this case, the deformation of valve body (540) that accompanies the closing of valve body (540) is accomplished by first and second valve body accommodating spaces (51a) and (52a) that are not joined with channel space (S), so the volume of channel space (S) does not change accompanying the closing of valve body (540). Thus, channel space (S) can be kept from reaching negative pressure by the closing of valve body (540).

## Claims

1. A connector (100, 200, 300, 400, 500) having a fluid inlet port (13) and a fluid outlet port (12), said connector including a valve (40) associated with the fluid inlet port (13) such that the connection of a liquid supply means (L) to said fluid inlet port causes the valve (40) to move to an open position, wherein said valve comprises two movable sealing bodies (42, 47) movable in respective accommodating chambers (51, 52) in response to pressure exerted by said liquid supply means (L), said valve further comprising said accomodating chambers (51, 52) which are separate from a fluid flow channel (5) and wherein when said valve is in a closed position, said sealing bodies (42, 47) abut one another, **characterized in that** said accommodating chambers (51, 52) include respective venting channels (51b, 52b).

2. The connector according to claim 1, wherein said sealing bodies are made of a resiliently deformable material.

3. The connector according to claim 1, wherein said sealing bodies have a base region (42c, 47c), a hemifrustroconical region (42b, 47b), a hemicylindrical region (42a, 47a) and a support arm (43, 48) attached to said hemicylindrical region.

4. The connector according to claim 3, wherein in said closed position, only said base regions (42c, 47c) are positioned within respective accommodating chambers (51, 52).

5. The connector of claim 1, further including a second valve (61, 62).

6. The connector of claim 5, wherein said second valve (61, 62) is a leaf valve positioned immediately adjacent said first valve (40).

7. The connector according to claim 1 wherein said two movable sealing bodies are connected together with a slit being formed in the movable sealing body at the connection.

8. The connector according to any preceding claim wherein said connector includes a second fluid inlet port (11).

9. The connector according to claim 8 wherein said connector includes a valve (20) for controlling the supply of fluid from said first and second fluid inlet ports (11, 13) to said outlet port (12).

10. The connector according to claim 8, wherein said outlet port (12) and said second fluid inlet port (11) are push-connectable to a fluid transport tube (T1, T2).

11. The connector according to any one of claims 1 to 7 wherein said fluid inlet port (12) is a luer connector port.

## Patentansprüche

1. Verbinder (100, 200, 300, 400, 500) mit einem Fluideinlassanschluss (13) und einem Fluidauslassanschluss (12), wobei der Verbinder ein Ventil (40) umfasst, das dem Fluideinlassanschluss (13) zugeordnet ist, so dass die Verbindung eines Flüssigkeitszufuhrmittels (L) mit dem Fluideinlassanschluss bewirkt, dass das Ventil (40) in eine geöffnete Position bewegt wird, wobei das Ventil zwei bewegliche Schließkörper (42, 47) umfasst, die in jeweiligen Aufnahmekammern (51, 52) als Reaktion auf einen Druck, der durch die Flüssigkeitszufuhrmittel (L) ausgeübt wird, beweglich sind, wobei das Ventil die Aufnahmekammern (51, 52), die von einem Fluidströmungskanal (5) getrennt sind, umfasst und wobei dann, wenn das Ventil in einer geschlossenen Position ist, die Schließkörper (42, 47) aneinander anliegen, **dadurch gekennzeichnet, dass** die Aufnahmekammern (51, 52) jeweilige Entlüftungskanäle (51b, 52b) aufweisen.

2. Verbinder nach Anspruch 1, wobei die Schließkörper aus einem elastisch verformbaren Material hergestellt sind.

3. Verbinder nach Anspruch 1, wobei die Schließkörper einen Basisbereich (42c, 47c), einen halbkegelstumpfförmigen Bereich (42b, 47b), einen halbzylindrischen Bereich (42a, 47a) und einen Tragarm (43, 48), der an dem halbzylindrischen Bereich befestigt ist, besitzen.

4. Verbinder nach Anspruch 3, wobei in der geschlossenen Position nur die Basisbereiche (42c, 47c) in jeweiligen Aufnahmekammern (51, 52) positioniert sind.

5. Verbinder nach Anspruch 1, der ferner ein zweites Ventil (61, 62) umfasst.

6. Verbinder nach Anspruch 5, wobei das zweite Ventil (61, 62) ein Klappenventil ist, das direkt benachbart zu dem ersten Ventil (40) positioniert ist.

7. Verbinder nach Anspruch 1, wobei die zwei beweglichen Schließkörper miteinander verbunden sind, wobei in dem beweglichen Schließkörper an der Verbindung ein Schlitz ausgebildet ist.

8. Verbinder nach einem vorhergehenden Anspruch, wobei der Verbinder einen zweiten Fluideinlassanschluss (11) umfasst.

9. Verbinder nach Anspruch 8, wobei der Verbinder ein Ventil (20) zum Steuern der Zufuhr von Fluid von dem ersten und dem zweiten Fluideinlassanschluss (11, 13) zu dem Auslassanschluss (12) umfasst.

10. Verbinder nach Anspruch 8, wobei der Auslassanschluss (12) und der zweite Fluideinlassanschluss (11) mit einem Fluidtransportrohr (T1, T2) durch Schieben verbindbar sind.

11. Verbinder nach einem der Ansprüche 1 bis 7, wobei der Fluideinlassanschluss (11) ein Luer-Verbinderanschluss ist.

## Revendications

1. Raccord (100, 200, 300, 400, 500) doté d'un orifice (13) d'entrée de fluide et d'un orifice (12) de sortie de fluide, ledit raccord comprenant une soupape (40) associée à l'orifice (13) d'entrée de fluide de telle sorte que le raccordement d'un moyen (L) d'alimentation en liquide audit orifice d'entrée de fluide fait passer la soupape (40) dans une position ouverte, ladite soupape comportant deux corps mobiles (42, 47) d'étanchéité, mobiles dans des chambres (51, 52) de logement respectives en réaction à une pression exercée par ledit moyen (L) d'alimentation en liquide, ladite soupape comportant en outre lesdites chambres (51, 52) de logement qui sont distinctes d'un conduit (S) d'écoulement de fluide et, lorsque ladite soupape 53 est en position fermée, lesdits corps (42, 47) d'étanchéité portant l'un contre l'autre, **caractérisé en ce que** lesdites chambres (51, 52) de logement comprennent des conduits (51b, 52b) d'aération respectifs.

2. Raccord selon la revendication 1, lesdits corps d'étanchéité étant constitués d'un matériau déformable élastiquement.

3. Raccord selon la revendication 1, lesdits corps d'étanchéité présentant une région (42c, 47c) de base, une région semi-tronconique (42b, 47b), une région semi-cylindrique (42a, 47a) et un bras porteur (43, 48) fixé à ladite région semi-cylindrique.

4. Raccord selon la revendication 3, **caractérisé en ce que**, dans ladite position fermée, seules lesdites régions (42c, 47c) de base sont positionnées à l'intérieur de chambres (51, 52) de logement respectives.

5. Raccord selon la revendication 1, comprenant en outre une deuxième soupape (61, 62).

6. Raccord selon la revendication 5, ladite deuxième soupape (61, 62) étant une soupape à lamelle positionnée au voisinage immédiat de ladite première soupape (40).

7. Raccord selon la revendication 1, lesdits deux corps mobiles d'étanchéité étant reliés entre eux, une fente étant formée dans le corps mobile d'étanchéité au niveau du raccordement.

8. Raccord selon l'une quelconque des revendications précédentes, ledit raccord comprenant un deuxième orifice (11) d'entrée de fluide.

9. Raccord selon la revendication 8, ledit raccord comprenant une soupape (20) servant à commander l'alimentation en fluide desdits premier et deuxième orifices (11, 13) d'entrée de fluide audit orifice (12) de sortie.

10. Raccord selon la revendication 8, ledit orifice (12) de sortie et ledit deuxième orifice (11) d'entrée de fluide pouvant être raccordés par enfoncement à un tube (T1, T2) de transport de fluide.

11. Raccord selon l'une quelconque des revendications 1 à 7, ledit orifice (11) d'entrée de fluide étant un orifice de raccord de Luer.
